# FASCICULE DE BREVET EUROPEEN

(11) **EP 1 525 246 B1**
(45) Date de publication et mention de la délivrance du brevet: **30.04.2014**
(21) Numéro de dépôt: 03755626.3
(22) Date de dépôt: 23.07.2003
(51) Int. Cl.: A61K 8/88, A61K 47/34, A61K 47/48, A61Q 19/00, C08G 69/10, C08G 69/48

(54) **POLYAMINOACIDES FONCTIONNALISES PAR AU MOINS UN GROUPEMENT HYDROPHOBE ET LEURS APPLICATIONS NOTAMMENT THERAPEUTIQUES**
MIT HYDROPHOBEN GRUPPEN FUNKTIONALISIERTE POLYAMINOSÄURE UND DEREN ANWENDUNGEN, INSBESONDERE IM THERAPEUTISCHEN BEREICH
POLYAMINO ACIDS FUNCTIONALIZED BY AT LEAST ONE HYDROPHOBIC GROUP AND THE THERAPEUTIC APPLICATIONS THEREOF

(30) Priorité: 30.07.2002 FR 0209670
(43) Date de publication de la demande: 27.04.2005
(73) Titulaire: FLAMEL TECHNOLOGIES, 69200 Vénissieux (FR)
(72) Inventeur: ANGOT, Stéphanie, 33200 BORDEAUX (FR); BREYNE, Olivier, F-69004 Lyon (FR); CHAN, You-Ping, F-69008 Lyon (FR); SOULA, Gérard, F-69330 Meyzieu (FR)
(74) Mandataire: Cabinet Plasseraud
(86) Numéro de dépôt international: PCT/FR2003/002329
(87) Numéro de publication internationale: WO 2004/013206

(56) Documents cités:
- WO-A-99/61512
- FR-A- 2 732 218
- US-B1- 6 320 017

## Description

La présente invention concerne des nouveaux matériaux à base de polyaminoacide biodégradables, utiles notamment pour la vectorisation de principe(s) actif(s) (PA).

L'invention vise aussi de nouvelles compositions pharmaceutiques, cosmétiques, diététiques ou phytosanitaires à base de ces polyaminoacides. Ces compositions peuvent être du type de celles permettant la vectorisation de PA et se présentant de préférence sous forme d'émulsions, de micelles, de particules, de gels, d'implants ou de films.

Les PA considérés sont, avantageusement, des composés biologiquement actifs et qui peuvent être administrés à un organisme animal ou humain par voie orale, parentérale, nasale, vaginale, oculaire, sous-cutanée, intraveineuse, intra-musculaire, intradermique, intrapéritonéale, intracérébrale ou buccale.

Les PA plus particulièrement mais non limitativement concernés par l'invention sont des protéines, des glycoprotéines, des peptides, des polysaccharides, des lipopolysaccharides, des oligo ou des polynucléotides, et des molécules organiques. Mais il peut aussi s'agir de produits cosmétiques ou de produits phytosanitaires, tels que des herbicides, des insecticides ou des fongicides.

Dans le domaine de la vectorisation des principes actifs notamment médicamenteux, il existe un besoin, dans beaucoup de cas :
- de les protéger contre la dégradation (hydrolyse, précipitation sur site, digestion enzymatique) jusqu'à ce qu'ils atteignent leur site d'action,
- et/ou de contrôler leur vitesse de libération afin de maintenir un niveau constant sur une durée définie, soit
- et/ou de véhiculer (en les protégeant) au site d'action.

A ces fins, plusieurs types de polymères ont été étudiés et certains sont même disponibles commercialement. On peut citer par exemple les polymères du type polylactique, polylactique-glycolique, polyoxyethylène-oxypropylène, polyaminoacide ou encore polysaccharide. Ces polymères constituent des matières premières permettant de fabriquer, par exemple, des implants massiques, des microparticules, des nanoparticules, des vésicules, des micelles ou des gels. Outre le fait que ces polymères doivent être adaptés à la fabrication de tels systèmes, ils doivent également être biocompatibles, non-toxiques, non-immunogènes, économiques et ils doivent pouvoir être facilement éliminés du corps et/ou biodégradables. Sur ce dernier aspect, il est de surcroît essentiel que la biodégradation dans l'organisme génère des produits non toxiques.

A titre d'illustration de l'art antérieur concernant des polymères employés comme matières premières pour la réalisation de systèmes de vectorisation de PA, divers brevets ou demandes de brevet ou articles scientifiques sont évoqués ci-après.

Le brevet US 4 652 441 décrit des microcapsules de polylactide encapsulant l'hormone LH-RH. Ces microcapsules sont produites en préparant une émulsion eau-dans-huile-dans-eau et comprennent une couche interne aqueuse contenant l'hormone, une substance (gélatine) fixant cette dernière, une couche huileuse de polylactide, ainsi qu'une couche externe aqueuse (Alcool polyvinylique). La libération du PA peut se faire sur une période de plus de deux semaines après injection sous-cutanée.

Le brevet US 6 153 193 décrit des compositions à base de micelles de poly(oxyéthylène)-poly(oxypropylène) amphiphiles, pour la vectorisation d'anti-cancéreux tel que l'adriamycine.

Akiyoshi et al. (J. Controlled Release 1998, 54, 313-320) décrivent des pullulans qui sont rendus hydrophobes par greffage de cholestérol et qui forment des nanoparticules dans l'eau. Ces nanoparticules aptes à se complexer de manière réversible avec l'insuline, forment des suspensions colloïdales stables.

Le brevet US 4 351 337 décrit des copolyaminoacides amphiphiles, à base de leucine et de glutamate, utilisables sous forme d'implants ou de microparticules pour la libération contrôlée de principes actifs. La libération de ces derniers peut se faire sur une durée très longue dépendant de la vitesse de dégradation du polymère.

Le brevet US 4 888 398 décrit des polymères à base de polyglutamate ou polyaspartate, et éventuellement polyleucine, avec des groupements pendants de type alkyloxycarbonylméthyle, placés de façon aléatoire sur la chaîne polyaminoacide. Ces polyaminoacides, greffés par des groupements latéraux e.g. méthoxycarbonylméthyle, sont utilisables sous forme d'implants biodégradables contenant un PA à libération prolongée.

Le brevet US 5 904 936 décrit des nanoparticules obtenues à partir d'un polymère bloc polyleucine-polyglutamate, aptes à former des suspensions colloïdales stables et capables de s'associer spontanément avec des protéines biologiquement actives sans les dénaturer. Ces dernières peuvent ensuite être libérées in vivo de manière contrôlée, sur une longue période.

Le brevet US 5 449 513 décrit des copolymères bloc amphiphiles comprenant un bloc polyoxyéthylène et un bloc polyaminoacide, par exemple poly(bêta-benzyl-L-aspartate). Ces polymères polyoxyéthylène-polybenzylaspartate forment des micelles qui sont aptes à encapsuler des molécules actives hydrophobes telles que l'adryamicine ou l'indométhacine.

La demande de brevet WO 99/61512 décrit des polylysines et des polyornithnes fonctionnalisées par un groupe hydrophobe (acide palmitique relié à la polylysine ou ornithine) et un groupe hydrophile (polyoxyéthylène). Ces polymères, par exemple la polylysine greffée avec des chaînes polyoxyéthylène et palmitoyle forment en présence de cholestérol des vésicules capables d'encapsuler la doxorubicine ou l'ADN. Ces polymères à base de polylysines sont cationiques en milieu physiologique.

La demande de brevet WO 00/30618, de la demanderesse, décrit des polymères blocs ou aléatoires poly(glutamate de sodium)(polyglutamate de méthyle, d'éthyle, d'hexa décyle ou de dodécyle), aptes à former des suspensions colloïdales stables et capables de s'associer spontanément avec des protéines biologiquement actives sans les dénaturer. Ces dernières peuvent ensuite être libérées in vivo de manière contrôlée, sur une longue période. Ces copolyaminoacides amphiphiles sont modifiés par la présence d'une chaîne latérale alkyle hydrophobe. Ces groupements alkyles sont greffés de façon covalente sur le polymère via une fonction ester, qui résulte de la réaction d'un alcool ou d'un iodoalcane, précurseur du greffon alcool, avec la fonction carboxylique de l'unité glutamique. Ces polymères sont anioniques en milieu physiologique.
Ils restent perfectibles à au moins deux égards, selon l'application visée :
⇒ la stabilité relative de la fonction ester dans un milieu aqueux,
⇒ et la mise en oeuvre comme précurseurs de greffons alkyles hydrophobes, de certains alcools non naturels tels que l'hexanol. Ce dernier aspect est notamment problématique en terme de toxicité si la quantité de polymère comportant des résidus de tels alcools devient importante.

Ainsi, même s'il existe de très nombreuses solutions techniques dans l'art antérieur, développées et proposées pour la vectorisation des principes actifs médicamenteux, la réponse à l'ensemble des exigences est difficile à obtenir et demeure insatisfaisante.

Dans ce contexte, l'un des objectifs essentiels de la présente invention est de fournir une nouvelle sous famille de polymères (de préférence anioniques) à base de polyglutamate et polyaspartate, qui représentent un perfectionnement par rapport à ceux décrits dans la demande de brevet WO-A-00/30618.

Un autre objectif essentiel de la présente invention est que ces polymères perfectionnés soient susceptibles d'être utilisés pour la vectorisation de PA et permettent de satisfaire de manière optimale à toutes les spécifications du cahier des charges :
○ biocompatibilité,
○ biodégradabilité,
○ stabilité à l'hydrolyse,
○ ces polymères étant eux-même propres :
   ■ à former aisément et économiquement des suspensions colloïdales aqueuses stables,
   ■ à s'associer facilement avec de nombreux principes actifs,
   ■ et à libérer ces principes actifs in vivo.

Cet objectif, parmi d'autres, est atteint par la présente invention qui concerne tout d'abord un polyaminoacide amphiphile comprenant des unités aspartiques et/ou des unités glutamiques, porteuses de greffons comportant chacun au moins un motif hydrophobe, caractérisé en ce qu'au moins une partie de ces greffons hydrophobes est liée aux unités aspartiques et/ou glutamiques, chacun par l'intermédiaire d'un *"espaceur"* impliqué dans et/ou comprenant au moins deux liaisons amides.

Selon l'invention, on sélectionne un motif hydrophobe issu d'un acide ou de l'un de ses dérivés, de préférence un acide gras naturel, puis on greffe cet acide hydrophobe sur le polymère via un *"espaceur"* de préférence à base de lysine ou d'ornithine. Les deux liaisons impliquées dans le greffage du groupement hydrophobe sont des amides (de la même nature que les liaisons peptidiques du polyaminoacide). Le concept inventif repose donc en partie sur l'utilisation d'acides hydrophobes comme précurseurs des greffons, au lieu des alcools ou des iodoalkyles connus, ce qui permet d'obtenir des polymères bien plus stables en milieu aqueux.

Ces nouveaux polymères ont un squelette biodégradable à base de polyaminoacide porteur de chaînes latérales comprenant un motif hydrophobe. Ces polymères présentent des propriétés d'association et/ou d'encapsulation surprenantes en comparaison avec des produits analogues et de plus, ils sont facilement dégradés en présence d'enzymes.

Il est du mérite de la demanderesse d'avoir eu l'idée de combiner, de façon tout à fait judicieuse et avantageuse, des polyaminoacides particuliers polyAsp et/ou polyGlu, biodégradables et anioniques avec des greffons comportant au moins un motif hydrophobe issu d'un acide et reliés au squelette polyAsp et/ou polyGlu par une rotule comprenant au moins deux liaisons amide, et plus précisément un "*espaceur*" à base de lysine (encore plus préférablement de la L-Lysine) ou d'ornithine.
Ces nouveaux (co)polymères se sont avérés être particulièrement bien adaptés pour la vectorisation de PA.

Au sens de l'invention le terme «*polyaminoacide*» couvre aussi bien les oligoaminoacides comprenant de 2 à 20 unités aminoacide que les polyaminoacides comprenant plus de 20 unités aminoacide.

De préférence, les polyaminoacides selon la présente invention sont des oligomères ou des homopolymères comprenant des unités récurrentes aminoacide glutamique ou aspartique ou des copolymères comprenant un mélange de ces deux types d'unités aminoacide. Les unités considérées dans ces polymères sont des acides aminés ayant la configuration D. L ou D, L et sont liées par leurs positions alpha ou gamma pour l'unité glutamate ou glutamique et alpha ou bêta pour l'unité aspartique on aspartate.

Les unités aminoacide préférées sont celles ayant la configuration L et une liaison de type alpha.

De manière plus préférée encore, les polyaminoacides selon l'invention répondent à la formule générale (**I**) suivante : 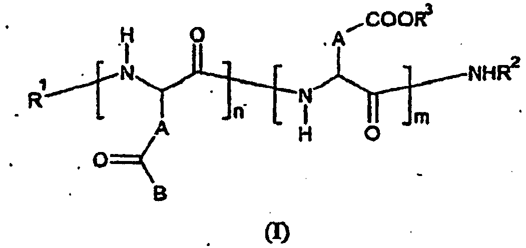 dans laquelle :
■ R¹ représente un H, un groupe acyle linéaire en C2 à C10 ou ramifié en C3 à C10, ou un pyruglutamate;
■ R² représente un H, un alkyle linéaire en C2 à C10 ou ramifié en C3 à C10, un benzyle, une unité acide aminé terminale;
■ R³ est un H ou une entité cationique, de préférence sélectionnée dans le groups comprenant :
   - les cations métalliques avantageusement choisis dans le sous-groupe comprenant : le sodium, le potassium, le calcium, le magnésium,
   - les cations organiques avantageusement choisis dans le sous-groupe comprenant:
      - les cations à base d'amine,
      - les cations à base d'oligoamine,
      - les cations à base de polyamine (la polyéthyléneimine étant particulièrement préférée),
      - les cations à base d'acide(s) aminé(s) avantageusement choisis dans la classe comprenant les cations à base de lysine ou d'arginine,
   - ou les polyaminoacides canoniques avantageusement choisis dans le sous-groupe comprenant la polylysine ou l'oligolysine;
■ les n groupements B représentent chacun indépendamment les uns des autres un radical monovalent de formule suivante : 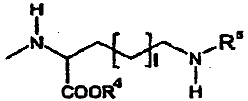 ou 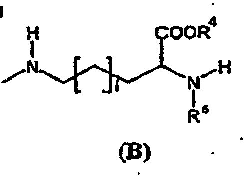 dans laquelle :
   - R⁴ représente indépendamment un groupement choisi dans la liste donnée pour R³, un alkyle linéaire en C1 à C6, un alkyle ramifié en C3 à C8 ou un benzyle;
   - R⁵ représente indépendamment un groupement choisi parmi des dérivés d'acides tels que ceux issus (i) d'alkyles saturés ou insakurés, linéaires en C2 à C24 ou ramifiés en C4 à C24 (ii) d'aryles, d'aralkyles ou d'alkylaryles en C7 à C24;
■ A représente indépendamment un -CH₂- (unité aspartique) ou -CH₂-CH₂-(unité glutamique);
■ n/(n+m) est défini comme le taux de greffage molaire et varie de 0,5 à 100 % molaire;
■ n + m varie de 3 à 1000, de préférence entre 30 et 300;
■ 1 est égale à 1 ("*espaceur*" ornithine) ou 2 ("*espaceur*" lysine).

De façon générale, les radicaux -R⁵ préférés sont des acyles issus d'acides gras naturels de préférence choisi dans le groupe comprenant l'acide palmitique, l'acide stéarique, l'acide oléique et l'acide linoléique.

Selon un premier mode de réalisation de l'invention, les polyaminoacides sont des homopolymères d'alpha-L-glutamate ou d'acide alpha-L-glutamique.

Selon un deuxième mode de réalisation de l'invention, les polyaminoacides sont des homopolymères d'alpha-L-aspartate ou d'acide alpha-L-aspartique.

Selon un troisième mode de réalisation de l'invention, les polyaminoacides sont des copolymères d'alpha-L-aspartate/alpha-L-glutamate ou d'acide alpha-L-aspartique/acide alpha-L-glutamique.

Avantageusement, la distribution des unités aspartiques et/ou glutamiques porteuses de greffons comportant au moins un motif hydrophobe est telle que les polymères ainsi constitués sont soit aléatoires, soit de type bloc, soit de type multibloc.

Selon un autre mode de définition, les polyaminoacides selon l'invention ont une masse molaire qui se situe entre 2000 et 100.000 g/mole, et de préférence entre 5000 et 40.000 g/mole.

Il est par ailleurs préférable que le taux de greffage molaire en motif hydrophobe des polyaminoacides selon l'invention, soit compris entre 2 et 50 %, et de préférence entre 5 et 20 %.

De manière remarquable, les polyaminoacides de l'invention sont susceptibles d'être utilisés de plusieurs façons selon le taux de greffage. Les méthodes de mise en forme d'un polymère pour l'encapsulation d'un principe actif sous les diverses formes visées par l'invention sont connues de l'homme de l'art. Pour plus de détails, on peut se référer, par exemple à ces quelques références particulièrement pertinentes :
"Microspheres, Microcapsules and Liposomes ; vol 1. Preparation and chemical applications" Ed. R. Arshady, Citus Books 1999. ISBN : 0-9532187-1-6.
"Sustained-Release Injectable Products" Ed. J. Senior et M. Radomsky, Interpharm Press 2000. ISBN : 1-57491-101-5.
"Colloidal Drug Delivery Systems" Ed. J. Kreuter, Marcel Dekker, Inc. 1994. ISBN : 0-8247-9214-9.
"Handbook of Pharmaceutical Controlled Release Technology" Ed. D.L. Wise, Marcel Dekker, Inc. 2000. ISBN : 0-8247-0369-3.

Les polyaminoacides sont en outre extrêmement intéressants, du fait qu'à un taux de greffage relativement faible de l'ordre de 3 à 10 %, ils se dispersent dans l'eau à pH 7,4 (par exemple avec un tampon phosphate) pour donner des solutions ou des suspensions colloïdales ou des gels, en fonction de la concentration de polymères et du taux de greffage. De plus, les polyaminoacides (sous forme de particules ou non), peuvent encapsuler ou s'associer aisément avec des principes actifs tels que des protéines, peptides ou petites molécules. La mise en forme préférée est celle décrite dans la demande de brevet WO 00/30618 de la demanderesse et qui consiste à disperser le polymère dans l'eau et à incuber la solution en présence d'un PA. Cette solution peut ensuite être filtrée sous 0,2 µm puis directement injectée à un patient

Au-delà de 10 % de taux de greffage, le polymère peut former des microparticules capables d'associer ou d'encapsuler des PA. Dans ce contexte, la mise en forme des microparticules peut se faire en co-solubilisant le PA et le polymère dans un solvant organique approprié puis le mélange est précipité dans l'eau. Les particules sont ensuite récupérées par filtration et peuvent ensuite être utilisées pour une administration par voie orale (sous forme de gélule, sous forme compactée et/ou enrobée ou bien encore sous forme dispersée dans une huile) ou par voie parentérale après redispersion dans l'eau.

A des taux supérieurs à 30 % de greffage, la redispersion du polymère en phase aqueuse devient plus difficile du fait de la quantité plus faible des fonctions carboxylate ionisables et le polymère précipite. Dans ce cas, le polymère peut être solubilisé dans un solvant biocompatible tel que la N-méthylpyrrolidone ou une huile appropriée telle que le Miglyol® puis injecté en intramusculaire ou sous-cutanée ou dans une tumeur. La X diffusion du solvant ou de l'huile conduit à la précipitation du polymère sur le site d'injection et forme ainsi un dépôt Ces dépôts assurent ensuite une libération contrôlée par diffusion et/ou par érosion et/ou par dégradation hydrolytique ou enzymatique du polymère.

De façon générale, les polymères de invention, sous forme neutre ou ionisée, sont utilisables seuls ou dans une composition liquide, solide ou gel et dans un milieu aqueux ou organique.

Il convient de comprendre que le polymère à base de polyaminoacides contient des fonctions carboxyliques qui sont soit neutres (forme COOH), soit ionisées selon le pH et la composition. Pour cette raison, la solubilité dans une phase aqueuse est directement fonction du taux de COOH libre du polymère (non greffé par le motif hydrophobe) et ceux de "*l'espaceur*" (COOR⁴ avec R⁴ égale H) et du pH. En solution aqueuse, le contre-cation peut être un cation métallique tel que le sodium, le calcium ou le magnésium, ou un cation organique tel que la triéthanolamine, le tris(hydroxyméthyl)-aminométhane ou une polyamine telle que la polyéthylèneimine.

Les polymères de l'invention sont obtenus par des méthodes connues de l'homme de l'art. Les polyaminoacides peuvent être obtenus par greffage de "*l'espaceur*" lysine ou ornithine, préalablement fonctionnalisé sur une des extrémités aminées par un groupement hydrophobe.

On prépare par exemple un polyaminoacide, homopolyglutamate, homopoly-aspartate ou un copolymère glutamate/aspartate, bloc, multibloc ou aléatoire selon des méthodes classiques.

Pour l'obtention de polyaminoacide de type alpha, la technique la plus courante est basée sur la polymérisation d'anhydrides de N-carboxy-aminoacides (NCA), décrite, par exemple, dans l'article "Biopolymers, 1976, 15, 1869 et dans l'ouvrage de H.R- Kricheldorf "alpha-Aminoacid N-carboxy Anhydride and related Heterocycles" Springer Verlag (1987). Les dérivés d'NCA sont de préférence des dérivés NCA-O-Me, NCA-O-Et ou NCA-O-Bz (Me = Méthyle, Et = Ethyle et Bz = Benzyle). Les polymères sont ensuite hydrolysés dans des conditions appropriées pour obtenir le polymère sous sa forme acide. Ces méthodes sont inspirées de la description donnée dans le brevet FR 2 801 226 de la demanderesse. Un certain nombre de polymères utilisables selon l'invention, par exemple, de type poly(acide alpha-L-aspartique), poly(acide alpha-L-glutamique), poly(acide alpha-D-glutamique) et poly(acide gamma-L-glutamique) de masses valables sont disponibles commercialement. Le polyaspartique de type alpha-béta est obtenu par condensation de l'acide aspartique (pour obtenir un polysuccinimide) suivie d'une hydrolyse basique (voir Tomida et al. Polymer 1997,38,4433-36).

Le greffon hydrophobe ayant une fonction amine peut être préparé selon l'une des voies proposées ci-dessous. A titre d'exemple, "*l'espaceur*" de type lysine est celui retenu pour illustrer la voie d'obtention.

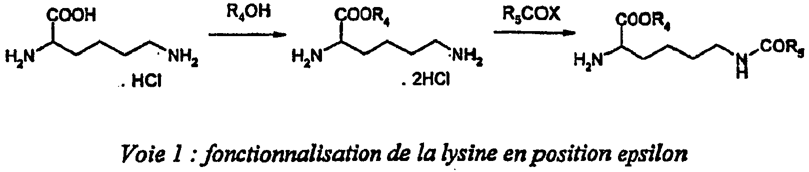

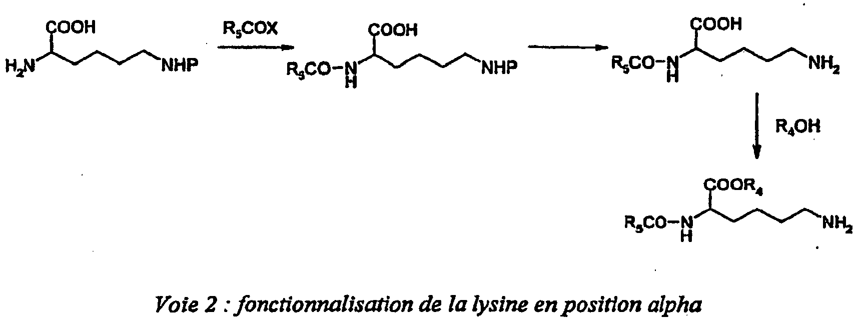

Dans ces schémas, X est un groupement partant tel qu'un halogénure ou un N-hydroxysuccinimide et P un groupement protecteur d'amine telle que le N-benzyloxycarbonyle (Cb) ou t-Boc. Pour une description de méthode générale ou complémentaire, on peut citer les deux références suivantes : US-B-4 126 628 et Volgler et al. Helv. Chim. Acta 1964, 47, 526-544*.*

Le couplage de l'amine ainsi obtenue avec une fonction acide du polymère est réalisé aisément par réaction du polyaminoacide en présence d'un carbodiimide comme agent de couplage et optionnellement, un catalyseur tel que le 4-dimethylaminopyridine et dans un solvant approprié tel que le diméthylformamide (DMF), la N-méthyl pyrrolidone (NMP) ou le diméthylsulfoxide (DMSO). Le carbodiimide est par exemple, le dicyclohexylcarbodiimide ou le diisopropylcarbodiimide. Le taux de greffage est contrôlé chimiquement par la stoechiométrie des constituants et réactifs ou le temps de réaction.

Selon un autre de ses aspects, l'invention vise une composition pharmaceutique, cosmétique, diététique ou phytosanitaire comprenant au moins un polyaminoacide tel que défini ci-dessus et éventuellement au moins un principe actif, qui peut être thérapeutique, cosmétique, diététique ou phytosanitaire.

De préférence, le principe actif est une protéine, une glycoprotéine, une protéine liée à une ou plusieurs chaînes polyalkylèneglycol {de préférence PolyÉthylèneGlycol (PEG) : "protéine-PEGylée"}, un polysaccharide, un lipopolysaccharide, un oligonucléotide, un polynucléotide ou un peptide.

Plus préférentiellement encore, le principe actif est une petite molécule organique hydrophobe, hydrophile ou amphiphile.

Cette composition peut être sous forme de nanoparticules, de microparticules, d'émulsions, de gels, de micelles, d'implants, de poudres ou de films.

Suivant l'une de ses formes particulièrement préférées, la composition, chargée ou non en principe actif(s), est une suspension colloïdale stable de nanoparticules et/ou de microparticules et/ou de micelles de polyaminoacides, dans une phase aqueuse.

La composition selon l'invention, dès lors qu'elle est pharmaceutique, peut être administrée par voie orale, parentérale, nasale, vaginale, oculaire, sous-cutanée, intraveineuse, intramusculaire, intradermique, intrapéritonéale, intracérébrale ou buccale.

Il est également envisageable que la composition soit sous forme de solution dans un solvant biocompatible, susceptible d'être injectée en sous-cutané, intramusculaire ou dans une tumeur.

Selon une autre variante, la composition selon l'invention est formulée de telle sorte qu'elle soit apte à former un dépôt sur le site d'injection.

L'invention vise aussi des compositions qui comprennent des polyaminoacides selon l'invention et des PA et qui sont susceptibles d'être utilisées pour la préparation :
- de médicaments, en particulier pour administration orale, nasale, vaginale, oculaire, sous-cutanée, intraveineuse, intramusculaire, intradermique, intrapéritonéale ou intracérébrale, les principes actifs de ces médicaments pouvant être, notamment, des protéines, des glycoprotéines, des protéines liées à une ou plusieurs chaînes polyalkylèneglycol {par exemple PolyÉthylèneGlycol (PEG), on parle alors de protéines "PEGylées"}, des peptides, des polysaccharides, des lipopolysaccharides, des oligonucléotides, des polynucléotides et des petites molécules organiques hydrophobes, hydrophiles ou amphiphiles ;
- et/ou des nutriments ;
- et/ou de produits cosmétiques ou phytosanitaires.

Cette préparation est caractérisée en ce qu'elle consiste essentiellement à mettre en oeuvre au moins l'un des polyaminoacides selon l'invention, tels qu'ils sont définis ci-dessus, et/ou la composition également décrite supra.

Comme indiqué ci-dessus, les techniques d'association d'un ou de plusieurs PA aux polyaminoacides greffés selon l'invention, sont décrites notamment dans la demande de brevet WO-A-00/30618.

L'invention concerne également une méthode de traitement thérapeutique consistant essentiellement à administrer la composition telle que décrite dans le présent exposé, par voie orale, parentérale, nasale, vaginale, oculaire, sous-cutanée, intraveineuse, intramusculaire, intradermique, intrapéritonéale, intracérébrale ou buccale.

L'invention concerne en outre une méthode de traitement thérapeutique consistant essentiellement à une composition telle que décrite supra sous forme de solution dans un solvant biocompatible puis de l'injecter en sous-cutané, intramusculaire ou dans une tumeur, de préférence de manière à ce qu'elle forme un dépôt sur le site d'injection.

Comme exemples de PA susceptibles d'être associés aux polyaminoacides selon l'invention, qu'ils soient ou non sous forme de (nano ou micro)particules, on peut citer :
○ les protéines telles que l'insuline, les interférons, les hormones de croissance, les interleukines, l'érythropoïétine ou les cytokines;
○ les peptides tels que le leuprolide ou la cyclosporine ;
○ les petites molécules telles que celles appartenant à la famille des anthracyclines, des taxoïdes ou des camptothécines ;
○ et leurs mélanges.

L'invention sera mieux comprise et ses avantages et variantes de mise en oeuvre ressortiront bien des exemples qui suivent et qui décrivent la synthèse des polyaminoacides greffés par un groupement hydrophobe, leur transformation en système de vectorisation de PA (suspension colloïdale aqueuse stable) et la démonstration de la capacité d'un tel système de s'associer à des PA (petites molécules organiques, protéines) pour former des compositions pharmaceutiques.

### Exemple 1 : Préparation du polymère P1

### Synthèse d'un polyglutamate greffé avec de l'acide palmitique via un "espaceur" lysine.

### 1/ Synthèse du greffon hydrophobe (LysC₁₆)

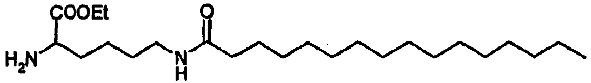

On ajoute 11,9 g de chlorure de thionyle à froid sur le chlorydrate de la L-lysine (11 g) dans 100 ml d'éthanol et on laisse 3h à reflux. Après évaporation de l'excès de chlorure de thionyle et d'éthanol, le produit est lavé par de l'heptane puis séché sous vide. On obtient 14,8 g de bischlorhydrate de lysine dont la fonction acide est estérifiée par l'éthanol. On fait réagir ce produit (4,9 g) avec 7 g du dérivé N-hydroxy-succinimide de l'acide palmitique (Rn Cas 14464-31-4, disponible de Sigma) dans un mélange acétone/eau (80 + 40 ml respectivement), en présence de 7 g de triéthylamine. Après une nuit à température ambiante, on précipite le produit par ajout d'acide chlorhydrique 1N. Après une purification par passage sur une colonne de silice, on récupère 3,3 g du produit souhaité. Sa structure est confirmée par spectroscopie RMN.

### 2/ Synthèse du polymère

On solubilise 4 g d'un alpha-L-polyglutamate (de masse équivalente à environ 10.000 par rapport à un standard en polyoxyéthylène et obtenue par polymérisation de monomères constitués par des dérivés N-CarboxyAnhydride de glutamate de méthyle : NCAGluOMe. Cette polymérisation est suivie d'une hydrolyse comme décrits dans la demande de brevet FR 2 801 226) dans 80 ml de DiMéthylFormamide (DMF) en chauffant à 40° C pendant 2 heures. Une fois le polymère solubilisé, on laisse revenir la température à 25° C et on ajoute successivement 1,025 g du greffon hydrophobe LysC16 préalablement solubilisé dans 6 ml de DMF, 0,06 g de 4-diméthylaminopyridine préalablement solubilisé dans 6 ml de DMF et 0,37 g de diisopropylcarbodiimide préalablement solubilisé dans 6 ml de DMF. Après 8 heures à 25° C sous agitation, le milieu réactionnel est versé dans 800 ml d'eau contenant 15 % de chlorure de sodium et d'acide chlorhydrique (pH 2). Le polymère précipité est ensuite récupéré par filtration, lavé par de l'acide chlorhydrique 0,1 N puis par de l'eau. Le polymère est ensuite solubilisé dans 75 ml de DMF puis précipité dans de l'eau contenant comme précédemment du sel et de l'acide à pH 2. Après deux lavages à l'eau, on lave plusieurs fois par de l'éther diisopropylique. Le polymère est ensuite séché à l'étuve sous vide à 40° C. On obtient un rendement de l'ordre de 95 %. Le taux de greffage estimé par RMN du proton est d'environ 8,1 %.

### Exemple 2 : Préparation du polymère P2

On prépare le polymère P2 dans les mêmes conditions que celles utilisées pour le polymère P1 sauf que le taux de greffage est diminué.

Le tableau ci-dessous rassemble les caractéristiques des deux polymères.

**Tableau 1**

| **Polymère** | **Greffon** | **Taux de greffage (RMN)** | **Mn* g/mole (équiv. PMMA)** |
|---|---|---|---|
| P1 | LysC₁₆ | **8,1 %** | 17800 |
| P2 | LysC₁₆ | **4,9 %** | 15900 |

| | | | |
|---|---|---|---|
| * Mn : masse molaire en nombre. Dans tous les cas, la quantité de lysine-C₁₆ effectivement greffée a été conformée par RMN. | | | |

### Exemple 3 : Analyse des polymères en solution aqueuse

Les polymères sont mis en solution dans un tampon phosphate salin à pH 7,4 à une concentration de 10 à 40 mg/ml et on ajuste le pH à 7,4 par ajout de soude à 0,1 N. On observe visuellement la solubilisation.

**Tableau 2 : solubilité dans l'eau saline à pH 7,4**

| **Polymère** | **Taux de greffage** | **Concentration** | **Aspect** |
|---|---|---|---|
| P1 | 8,1 % | 10 mg/ml | Soluble et limpide |
| P2 | 4,9 % | 10 mg/ml | Soluble et limpide |

| | | | |
|---|---|---|---|
| Tampon phosphate : 0,01 M phosphate, 0,0027 M KCl et 0,137M NaCl.. | | | |

Une analyse des solutions à environ 1 mg/ml par diffraction de lumière (488 nm) révèle la présence d'objets nanométriques de l'ordre de 100 nm pour le polymère P1 et l'absence d'objets pour le polymère P2 (absence de diffraction de la lumière).

### Exemple 4 : Stabilité aqueuse du polymère P1

Pour cette étude, le polymère P1 a été comparé avec un polymère analogue ayant une chaîne hexadécanol greffé sur un polyglutamate via une fonction ester (polymère C1 ; taux de greffage de 9,5 % molaire). La synthèse de ce type de polymère analogue est décrite dans le brevet WO-A-00/30618.

Dans cette étude, des conditions « accélérées » ont été mises en oeuvre : les deux polymères ont été placés à une concentration de 30 g/L, à un pH de 10 et à 60°C, pendant 5 jours. Les polymères sont ensuite analysés par RMN après précipitation préalable par HCl 1N et lavages par de l'éther diisopropylique. Les résultats sont donnés dans le tableau 3 ci-dessous.

**Tableau 3**

| **Polymère** | **Taux de greffage t = 0** | **Taux de greffage t = 5 j** |
|---|---|---|
| P1 | 8,1 % | 8,1 % |
| C1* | 9,5 % | 4,3 % |

| | | |
|---|---|---|
| * WO-A-00/30618 | | |

Ces résultats montrent clairement que le polymère P1 conserve l'intégralité de ses groupements hydrophobes Lys-C₁₆ dans des conditions élevées de pH et de température. En revanche, son analogue en C₁₆ subit une perte de plus de 50 % de ces groupements alkyles hydrophobes.

### Exemple 5 : Adsorption d'un colorant sur le polymère P1 et P2

Selon l'un des objets de l'invention, les polymères peuvent être utilisés dans l'eau et associés ou encapsuler un principe actif (sous forme de suspension colloïdale ou non). Pour cette application, il est démontré dans l'expérience ci-après que les polymères P1 et P2 sont capables d'associer ou encapsuler un colorant modèle.

L'étude est réalisée de la façon suivante : on solubilise les polymères dans une solution aqueuse à pH 7 (tampon phosphate) et on ajoute 5 mg du colorant dénommé "Orange OT" (Rn CAS : 2646-17-5). On laisse les solutions dans un bain d'ultrason pendant une heure pour réaliser l'association. Les solutions sont ensuite centrifugées pour éliminer le colorant non associé et on mesure la densité optique (DO) au λmax du colorant (495 nm), après dilution.

**Tableau 4**

| **Polymère** | **Taux de greffage** | **Concentration polymère** | **DO induite** |
|---|---|---|---|
| P1 | 8,1 % molaire | 13 mg/ml | 0,15 |
| P2 | 4,9 % molaire | 20 mg/ml | 0,25 |
| Polyglutamate | 0 % molaire | 25 mg/ml | 0,016 |

### Exemple 6 : Association des polymères avec l'insuline

On prépare une solution aqueuse contenant 10 mg de polymère par millilitre à pH 7,4 et 200 UI d'insuline (7,4 mg). On laisse incuber les solutions pendant deux heures à température ambiante et on sépare l'insuline libre de l'insuline associée par ultrafiltration (seuil à 100 KDa, 15 minutes sous 10000G à 18° C). L'insuline libre récupérée dans le filtrat est ensuite dosée par CLHP (Chromatographie Liquide Haute Performance) et l'on déduit la quantité d'insuline associée.

Les résultats démontrent que les deux polymères P1 et P2 sont capables d'associer l'insuline à un taux supérieur à 190 IU (7,2 mg) pour 10 mg de polymère.

## Revendications

1. Polyaminoacide comprenant des unités aspartiques et/ou des unités glutamiques, porteuses de greffons comportant chacun au moins un motif hydrophobe, **caractérisé en ce qu'**au moins une partie de ces greffons hydrophobes sont liés aux unités aspartiques et/ou glutamiques, chacun par l'intermédiaire d'un "*espaceur*" et **caractérisé en ce que** le polyaminoacide a la formule générale **(I)** suivante : 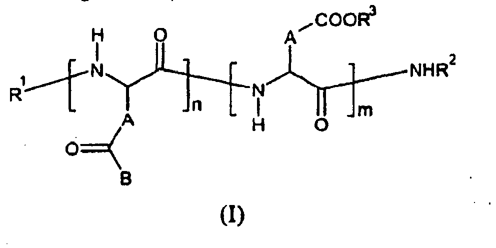 dans laquelle :
■ R¹ représente un H, un groupe acyle linéaire en C2 à C10 ou ramifié en C3 à C10, ou un pyroglutamate;
■ R² représente un H, un alkyle linéaire en C2 à C10 ou ramifié en C3 à C10, un benzyle, une unité acide aminé terminale;
■ R³ est un H ou une entité cationique sélectionnée dans le groupe comprenant :
- les cations métalliques,
- les cations organiques choisis dans le sous-groupe comprenant :
• les cations à base d'amine,
• les cations à base d'oligoamine,
• les cations à base de polyamine,
• les cations à base d'acide(s) aminé(s) choisis dans la classe comprenant les cations à base de lysine ou d'arginine,
- ou les polyaminoacides cationiques choisis dans le sous-groupe comprenant la polylysine ou l'oligolysine;
■ les n groupements B représentent chacun indépendamment les uns des autres un radical monovalent de formule suivante : 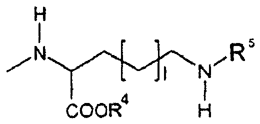 ou 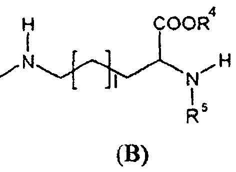 dans laquelle :
- R⁴ représente indépendamment un groupement choisi dans la liste donnée pour R³, un alkyle linéaire en C1 à C6, un alkyle ramifié en C3 à C8 ou un benzyle;
- R⁵ représente indépendamment un groupement hydrophobe choisi parmi un acide gras naturel ou l'un de ses dérivés;
■ A représente indépendamment un -CH₂- (unité aspartique) ou -CH₂-CH₂-(unité glutamique);
■ n/(n+m) est défini comme le taux de greffage molaire et varie de 0,5 à 100 % molaire;
■ n + m varie de 3 à 1000;
■ 1 est égale à 1 ("*espaceur*" ornithine) ou 2 ("*espaceur*" lysine).

2. Polyaminoacide selon la revendication 1, **caractérisé en ce que** R⁵ représente indépendamment un acyle d'acide gras naturel.

3. Polyaminoacide selon la revendication 1, **caractérisé en ce que** R⁵ représente indépendamment un acyle d'acide gras naturel choisi dans le groupe comprenant l'acide palmitique, l'acide stéarique, l'acide oléique et l'acide linoléique.

4. Polyaminoacide selon la revendication 1 **caractérisé en ce que** "*l'espaceur*" est dérivé de la L-lysine.

5. Polyaminoacide selon la revendication 1, **caractérisé en ce qu'**il est constitué d'un homopolymère d'alpha-L-glutamate ou d'acide alpha-L-glutamique, d'un homopolymère d'alpha-L-aspartate ou d'acide alpha-L-aspartique ou d'un copolymère d'alpha-L-aspartate/alpha-L-glutamate ou d'acide alpha-L-aspartique/acide alpha-L-glutamique.

6. Polyaminoacide selon la revendication 1, **caractérisé en ce que** la distribution des unités aspartiques et/ou glutamiques porteuses de greffons hydrophobes est telle que les polymères ainsi constitués sont soit aléatoires, soit de type bloc, soit de type multibloc.

7. Polyaminoacide selon la revendication 1, **caractérisé en ce que** sa masse molaire se situe entre 2000 et 100.000 g/mole.

8. Polyaminoacide selon la revendication 1, **caractérisé en ce que** le taux de greffage molaire se situe entre 2 et 50 %.

9. Composition pharmaceutique, cosmétique, diététique ou phytosanitaire comprenant au moins un polyaminoacide selon la revendication 1.

10. Composition selon la revendication 9 **caractérisée en ce qu'**elle comprend au moins un principe actif.

11. Composition selon la revendication 10, **caractérisée en ce que** le principe actif est une protéine, une glycoprotéine, une protéine liée à une ou plusieurs chaînes polyalkylèneglycol, un polysaccharide, un lipopolysaccharide, un oligonucléotide, un polynucléotide ou un peptide.

12. Composition selon la revendication 10, **caractérisée en ce que** le principe actif est une petite molécule organique hydrophobe, hydrophile ou amphiphile.

13. Composition selon la revendication 9 ou 10, **caractérisée en ce qu'**elle peut être administrée par voie orale, parentérale, nasale, vaginale, oculaire, sous-cutanée, intraveineuse, intramusculaire, intradermique, intra péritonéale, intracérébrale ou buccale.

14. Composition selon la revendication 9 ou 10, **caractérisée en ce qu'**elle est sous forme d'un gel, d'une émulsion, de micelles, de nanoparticules, de microparticules, d'une poudre ou d'un film.

15. Composition selon la revendication 9 ou 10, **caractérisée en ce qu'**elle est une suspension colloïdale de nanoparticules et/ou de microparticules et/ou de micelles de polyaminoacides, dans une phase aqueuse.

16. Composition selon la revendication 9 ou 10, **caractérisée en ce qu'**elle est sous forme de solution dans un solvant biocompatible et **en ce qu'**elle peut être injectée en sous-cutané, intramusculaire ou dans une tumeur.

17. Composition selon la revendication 9 ou 10, **caractérisée en ce qu'**elle est apte à former un dépôt sur le site d'injection.

18. Procédé de préparation :
• de médicaments pour administration orale, nasale, vaginale, oculaire, sous-cutanée, intraveineuse, intramusculaire, intradermique, intrapéritonéale ou intracérébrale, les principes actifs de ces médicaments étant des protéines, des glycoprotéines, des protéines liées à une ou plusieurs chaînes polyalkylèneglycol, des peptides, des polysaccharides, des lipopolysaccharides, des oligonucléotides, des polynucléotides et des petites molécules organiques hydrophobes, hydrophiles ou amphiphiles ;
• et/ou des nutriments;
• et/ou de produits cosmétiques ou phytosanitaires;
**caractérisé en ce qu'**il consiste essentiellement à mettre en oeuvre au moins un polyaminoacide selon la revendication 1 et/ou la composition selon la revendication 9 ou 10.

## Patentansprüche

1. Polyaminosäure mit Asparaginsäure- und/oder Glutaminsäure-Einheiten, die Pfropfanteile mit jeweils mindestens einer hydrophoben Einheit tragen, **dadurch gekennzeichnet, dass** mindestens ein Teil dieser hydrophoben Pfropfanteile jeweils über einen "Spacer" an die Asparaginsäure- und/oder Glutaminsäure-Einheiten gebunden ist, und **dadurch gekennzeichnet, dass** die Polyaminosäure die folgende Formel (I) aufweist: 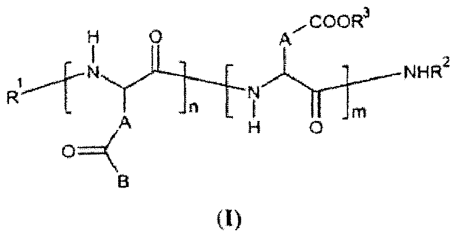 worin:
■ R¹ für H, eine lineare C₂- bis C₁₀- oder verzweigte C₃-C₁₀-Acylgruppe oder ein Pyroglutamat steht;
■ R² für H, lineares C₂- bis C₁₀- oder verzweigtes C₃-C₁₀-Alkyl, ein Benzyl oder eine endständige Aminosäure-Einheit steht;
■ R³ für H oder eine kationische Einheit steht, die unter
- Metallkationen,
- organischen Kationen, die aus der Untergruppe umfassend:
• Kationen auf Aminbasis,
• Kationen auf Oligoaminbasis,
• Kationen auf Polyaminbasis,
• Kationen auf Aminosäurebasis, die aus der Klasse umfassend Kationen auf Basis von Lysin oder Arginin ausgewählt sind,
ausgewählt sind,
- oder kationischen Polyaminosäuren, die aus der Untergruppe umfassend Polylysin oder Oligolysin ausgewählt sind,
ausgewählt ist,
■ die n Gruppen B jeweils unabhängig voneinander für einen einwertigen Rest der folgenden Formel: 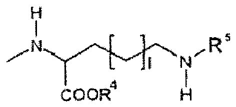 oder 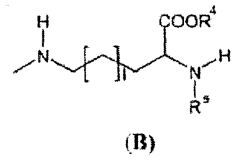 stehen, worin:
- R⁴ unabhängig für eine aus der für R³ angegebenen Liste ausgewählte Gruppe, ein lineares C₁- bis C₆-Alkyl, ein verzweigtes C₃-C₈-Alkyl oder ein Benzyl steht;
- R⁵ unabhängig für eine hydrophobe Gruppe steht, die unter einer natürlichen Fettsäure oder einem Derivat davon ausgewählt ist;
■ A unabhängig für -CH₂- (Asparaginsäure-Einheit) oder -CH₂₋CH₂- (Glutaminsäure-Einheit) steht;
■ n/ (n+m) als molarer Pfropfgrad definiert ist und von 0,5 bis 100 Mol-% variiert;
■ n + m von 3 bis 1000 variiert:
■ 1 gleich 1 (Ornithin-"Spacer") oder 2 (Lysin-"Spacer") ist.

2. Polyaminosäure nach Anspruch 1, **dadurch gekennzeichnet, dass** R⁵ unabhängig für ein Acyl einer natürlichen Fettsäure steht.

3. Polyaminosäure nach Anspruch 1, **dadurch gekennzeichnet, dass** R⁵ unabhängig für ein Acyl einer natürlichen Fettsäure, die aus der Gruppe umfassend Palmitinsäure, Stearinsäure, Ölsäure und Linolsäure ausgewählt ist, steht.

4. Polyaminosäure nach Anspruch 1, **dadurch gekennzeichnet, dass** der "Spacer" von L-Lysin abgeleitet ist.

5. Polyaminosäure nach Anspruch 1, **dadurch gekennzeichnet, dass** sie aus einem alpha-L-Glutamat- oder alpha-L-Glutaminsäure-Homopolymer, einem alpha-L-Aspartat- oder alpha-L-Asparaginsäure-Homopolymer oder einem alpha-L-Aspartat/alpha-L-Glutamat- oder alpha-L-Asparaginsäure-/alpha-L-Glutaminsäure-Copolymer besteht.

6. Polyaminosäure nach Anspruch 1, **dadurch gekennzeichnet, dass** hydrophobe Pfropfanteile tragende Asparaginsäure- und/oder Glutaminsäure-Einheiten so verteilt sind, dass die so gebildeten Polymere statistisch, blockartig oder multiblockartig aufgebaut sind.

7. Polyaminosäure nach Anspruch 1, **dadurch gekennzeichnet, dass** ihre Molmasse zwischen 2000 und 100.000 g/mol liegt.

8. Polyaminosäure nach Anspruch 1, **dadurch gekennzeichnet, dass** der molare Pfropfgrad zwischen 2 und 50% liegt.

9. Pharmazeutische, kosmetische, diätetische oder phytosanitäre Zusammensetzung, die mindestens eine Polyaminosäure nach Anspruch 1 umfasst.

10. Zusammensetzung nach Anspruch 9, **dadurch gekennzeichnet, dass** sie mindestens einen Wirkstoff umfasst.

11. Zusammensetzung nach Anspruch 10, **dadurch gekennzeichnet, dass** es sich bei dem Wirkstoff um ein Protein, ein Glykoprotein, ein an eine oder mehrere Polyalkylenglykolketten gebundenes Protein, ein Polysaccharid, ein Lipopolysaccharid, ein Oligonucleotid, ein Polynucleotid oder ein Peptid handelt.

12. Zusammensetzung nach Anspruch 10, **dadurch gekennzeichnet, dass** es sich bei dem Wirkstoff um ein kleines hydrophobes, hydrophiles oder amphiphiles organisches Molekül handelt.

13. Zusammensetzung nach Anspruch 9 oder 10, **dadurch gekennzeichnet, dass** sie auf oralem, parenteralem, nasalem, vaginalem, okulärem, subkutanem, intravenösem, intramuskulärem, intradermalem, intraperitonealem, intrazerebralem oder bukkalem Weg verabreichbar ist.

14. Zusammensetzung nach Anspruch 9 oder 10, **dadurch gekennzeichnet, dass** sie in Form eines Gels oder einer Emulsion, in Form von Micellen, Nanopartikeln oder Mikropartikeln oder in Form eines Pulvers oder eines Films vorliegt.

15. Zusammensetzung nach Anspruch 9 oder 10, **dadurch gekennzeichnet, dass** es sich dabei um eine kolloidale Suspension von Nanopartikeln und/oder Mikropartikeln und/oder Micellen der Polyaminosäuren in einer wässrigen Phase handelt.

16. Zusammensetzung nach Anspruch 9 oder 10, **dadurch gekennzeichnet, dass** sie in Form einer Lösung in einem biokompatiblen Lösungsmittel vorliegt und auf subkutanem oder intramuskulärem Wege oder in einen Tumor injizierbar ist.

17. Zusammensetzung nach Anspruch 9 oder 10, **dadurch gekennzeichnet, dass** sie am Injektionsort ein Depot bilden kann.

18. Verfahren zur Herstellung von:
• Arzneimitteln zur oralen, nasalen, vaginalen, okulären, subkutanen, intravenösen, intramuskulären, intradermalen, intraperitonealen oder intrazerebralen Verabreichung, wobei es sich bei den Wirkstoffen dieser Arzneimittel um Proteine, Glykoproteine, an eine oder mehrere Polyalkylenglykolketten gebundene Proteine, Peptide, Polysaccharide, Lipopolysaccharide, Oligonucleotide, Polynucleotide und kleine hydrophobe, hydrophile oder amphiphile organische Moleküle handelt;
• und/oder Nährstoffen
• und/oder kosmetischen oder phytosanitären Produkten;
**dadurch gekennzeichnet, dass** man dabei im Wesentlichen mindestens eine Polyaminosäure nach Anspruch 1 und/oder die Zusammensetzung nach Anspruch 9 oder 10 verwendet.

## Claims

1. Polyamino acid comprising aspartic units and/or glutamic units carrying grafts, each of which contains at least one hydrophobic unit, **characterized in that** at least some of these hydrophobic grafts are bonded to the aspartic and/or glutamic units, each by way of a "*spacer*" and **characterized in that** the polyamino acid has the general formula (I) below: 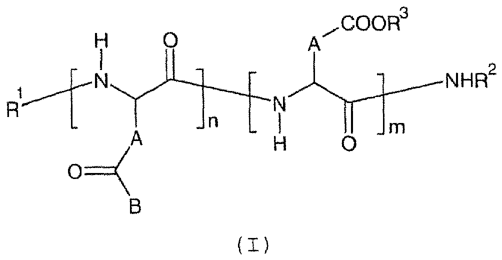 in which:
■ R¹ is a H, a linear C2 to C10 acyl or branched C3 to C10 acyl group or a pyroglutamate;
■ R² is a H, a linear C2 to C10 alkyl or branched C3 to C10 alkyl, a benzyl or a terminal amino acid unit;
■ R³ is a H or a cationic entity preferably selected from the group comprising:
- metal cations,
- organic cations selected from the subgroup comprising:
• cations based on amine,
• cations based on oligoamine,
• cations based on polyamine,
• and cations based on amino acid(s) chosen from the class comprising cations based on lysine or arginine,
- or cationic polyamino acids chosen from the subgroup comprising polylysine and oligolysine;
■ the n groups B independently of one another are each a monovalent radical of the following formula: 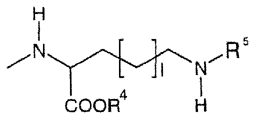 ou 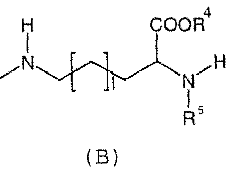 in which:
- R⁴ independently is a group selected from the list given for R³, a linear C1 to C6 alkyl, a branched C3 to C8 alkyl or a benzyl;
- R⁵ independently is a hydrophobic group selected from a natural fatty acid or one of its derivatives;
■ A independently is -CH₂- (aspartic unit) or -CH₂-CH₂- (glutamic unit);
■ n/(n + m) is defined as the molar grafting rate and varies from 0.5 to 100 mol%;
■ n + m varies from 3 to 1000;
■ and 1 is equal to 1 (ornithine "spacer") or 2 (lysine "*spacer*").

2. Polyamino acid according to claim 1, **characterized in that** R⁵ independently is an acyl of a natural fatty acid.

3. Polyamino acid according to claim 1, **characterized in that** R⁵ independently is an acyl of a natural fatty acid selected from the group comprising palmitic acid, stearic acid, oleic acid and linoleic acid.

4. Polyamino acid according to claim 1, **characterized in that** the "*spacer*" is derived from L-lysine.

5. Polyamino acid according to claim 1, **characterized in that** it consists of an alpha-L-glutamate or alpha-L-glutamic acid homopolymer or an alpha-L-aspartate or alpha-L-aspartic acid homopolymer or an alpha-L-aspartate/alpha-L-glutamate or alpha-L-aspartic acid /alpha-L-glutamic acid copolymer.

6. Polyamino acid according to claim 1, **characterized in that** the distribution of the aspartic and/or glutamic units carrying hydrophobic grafts is such that the resulting polymers are either random or of the block type or of the multiblock type.

7. Polyamino acid according to claim 1, **characterized in that** its molecular weight is between 2000 and 100,000 g/mol.

8. Polyamino acid according to claim 1, **characterized in that** the molar grafting rate is between 2 and 50%.

9. Pharmaceutical, cosmetic, dietetic or phytosanitary composition comprising at least one polyamino acid according to claim 1.

10. Composition according to claim 9, **characterized in that** it comprises at least one active principle.

11. Composition according to claim 10, **characterized in that** the active principle is a protein, a glycoprotein, a protein bonded to one or more polyalkylene glycol chains, a polysaccharide, a lipopolysaccharide, an oligonucleotide, a polynucleotide or a peptide.

12. Composition according to claim 10, **characterized in that** the active principle is a hydrophobic, hydrophilic or amphiphilic organic small molecule.

13. Composition according to claim 9 or 10, **characterized in that** it can be administered by the oral, parenteral, nasal, vaginal, ocular, subcutaneous, intravenous, intramuscular, intradermal, intra peritoneal, intracerebral or buccal route.

14. Composition according to claim 9 or 10, **characterized in that** it is in the form of a gel, an emulsion, micelles, nanoparticles, microparticles, a powder or a film.

15. Composition according to claim 9 or 10, **characterized in that** it is a colloidal suspension of nanoparticles and/or microparticles and/or micelles of polyamino acids in an aqueous phase.

16. Composition according to claim 9 or 10, **characterized in that** it is in the form of a solution in a biocompatible solvent and **in that** it can be injected by the subcutaneous or intramuscular route or into a tumor.

17. Composition according to claim 9 or 10, **characterized in that** it is capable of forming a deposit at the injection site.

18. Process for the preparation of:
• drugs, particularly for administration by the oral, nasal, vaginal, ocular, subcutaneous, intravenous, intramuscular, intradermal, intraperitoneal or intracerebral route, it being possible in particular for the active principles of these drugs to be proteins, glycoproteins, proteins bonded to one or more polyalkylene glycol chains, peptides, polysaccharides, lipopolysaccharides, oligonucleotides, polynucleotides and hydrophobic, hydrophilic or amphiphilic organic small molecules;
• and/or nutriments;
• and/or cosmetic or phytosanitary products, **characterized in that** it consists essentially in using at least one polyamino acid according to claim 1 and/or the composition according to claim 9 or 10.
